Europäisches Patentamt

**European Patent Office**

**Office européen des brevets**

(11) Publication number: **0 121 400**
**A2**

(12) **EUROPEAN PATENT APPLICATION**

(21) Application number: **84302075.1**

(22) Date of filing: **27.03.84**

(51) Int. Cl.³: **C 12 Q 1/24**
**C 12 N 5/00, C 12 N 11/04**
**//C12N15/00, C12P21/00**

(30) Priority: **30.03.83 US 480293**

(43) Date of publication of application:
**10.10.84 Bulletin 84/41**

(84) Designated Contracting States:
**CH DE FR GB IT LI**

(71) Applicant: **BIO-RESPONSE INC.**
**550 Ridgefield Road**
**Wilton Connecticut 06897(US)**

(72) Inventor: **Rose, Sam**
**2701 Claremont Boulevard**
**Berkeley California 94705(US)**

(74) Representative: **Marsh, Roy David et al,**
**Brewer & Son 5-9, Quality Court Chancery Lane**
**London WC2A 1HT(GB)**

(54) **Process for preparing and isolating living hybrid cells.**

(57) A method for isolating living, isolatable hybrid cells of a continuous dividing cell (e.g. a malignant myeloma cell) and a normal cell (incapable of continuous division) wherein hybridization is conducted in discrete, individual reaction chambers containing a predetermined number of continuous dividing cells and normal cells such that only a single hybrid therebetween can form in any chamber. The method insures that, after suitable isolation procedures, each identified living hybridoma necessarily will have been derived from a single hybrid cell of a continuous dividing cell and a normal cell and, thus, is capable of producing only a single, monoclonal product.

Croydon Printing Company Ltd.

-1-

PROCESS FOR PREPARING AND
ISOLATING LIVING HYBRID CELLS

The present invention relates to the production of a living, isolatable hybrid between two living cells, preferably between a malignant cell and a normal cell and, more particularly, to a process for producing monoclonal antibodies using the technique of hybridization.

In recent years, the art has developed the technique known as hybridization wherein a so-called hybrid cell is formed from two similar or dissimilar cell parents such that the hybrid cell exhibits desired characteristics of each of the parent cells. Typically, one of the chosen cell parents is a cell capable of continuous growth and division, such as a malignant (e.g., myeloma) cell, the intent being that the hybrid cell itself will exhibit the desirable characteristic of continuous division, thereby resulting in the growth of a homogeneous hybrid cell population (called a "hybridoma").

The other parent of the hybrid cell is a so-called "normal" cell exhibiting some desirable characteristic, but which is incapable of continuous division. The desirable characteristic of the normal cell typically is the production of a product such as an antibody. The hybrid between a continuous dividing cell and this normal, antibody-producing cell ideally exhibits the antibody production capability of its normal parent and the continuous division characteristic of its other parent, thereby constituting a cell which has the potential for producing large amounts of a desired antibody. For ease of reference in the following discussion, the continuous dividing cell is taken, for illustrative purposes, to be a malignant myeloma cell.

Hybridization of a malignant (myeloma) cell and an antibody-producing cell is used in the preparation of so-called "monoclonal" antibodies and, indeed, the desire to prepare such antibodies in large part was the impetus behind development of the hybridization technique. A hybrid of a myeloma cell and an antibody-producing cell will produce only a single (monoclonal) product since the hybrid is made up of only a single antibody-producing cell and this single antibody-producing cell is itself capable of producing only a single type of product. Thus, when a population of myeloma cells is mixed under hybridization conditions with a population of antibody-producing cells (producing in toto many different products or antibodies), hybrids will form from a myeloma cell and a single antibody-producing cell. Each individual hybrid can then be grown up to a degree sufficient to produce a quantity of its one particular product, and this product can then be tested to determine whether it is, e.g., an antibody to a particular antigen.

The efficacy of the hybridization technique in forming usable hybrids (such as those producing antibody) depends to a large degree on the ability to first isolate all hybrids between myeloma and normal cells from other cells in the mixed populations of cells, and thereafter, in a second step, to isolate from those hybrids just those producing a specific desired antibody. Thus, for example, when a population of myeloma cells is mixed, under hybridization conditions, with a population of normal cells (producing antibody), the resultant population may contain the following cells:

    (a)  hybrid cells between a myeloma cell and a normal cell;

    (b)  hybrid cells between two myeloma cells;

    (c)  hybrid cells between two normal cells;

    (d)  unhybridized normal cells; and

    (e)  unhybridized myeloma cells.

In the first isolation, all and only the cells of category (a) are desired. The cells of categories (a), (b) and (e) can be "isolated" from those of categories (c) and (d) because, with time, the normal cells eventually will die since they are incapable of continuous division. However, the isolation of the cells of category (a) from those of categories (b) and (e) is more difficult since all such cells are capable of continuous division. To accomplish the isolation, the art resorts to the use of so-called "mutant" myeloma cells as the myeloma cell population in the hybridization process. Thus, for use in the hybridization process, mutant myeloma cells are employed which possess a genetic deficiency in a material required for life in a particular medium. After mixing these cells and normal cells and effecting hybridization, the resultant cells are suspended in the above-noted medium. The unhybridized mutant myeloma

cells and hybrids of two mutant myeloma cells eventually die in this medium while the desired hybrids of mutant myeloma cells and normal cells survive because the genetic deficiency of the mutant myeloma is provided by the normal cell component of the hybrid cell.

A specific example of this isolation method is the use of mutant myeloma cells deficient in hypoxanthine phosphoribosyltransferase (HPRT). The growth of such cells is inhibited by selective hypoxanthine-aminopterin-thymidine (HAT) medium and, thus, suspension of all cells in this medium will result in the selective growth only of those mutant myeloma cells which have hybridized to a normal cell.

Another method for achieving the isolation of hybrids of myeloma and normal cells from all other cells present after hybridization is disclosed in my co-pending U.S. patent application, Serial No. 482,240, filed April 5, 1983 wherein, instead of using mutant myeloma cells in the hybridization process, there is employed a population of myeloma cells which have been chemically treated to inactivate a component thereof necessary for life in a particular medium.

After this first isolation, the only remaining living cells are hybrids between a myeloma cell and a normal cell. Because of the time required to effect the first isolation (i.e., to achieve the death of all cells other than the myeloma/normal hybrids), any such remaining hybrid cells may already have begun to divide and sub-divide. If it were an absolute certainty that the hybridization process resulted in the production of only one single hybrid between a myeloma cell and a normal cell, all that would remain to be done would be to permit the already started division and growth to continue (resulting in a homogeneous hybridoma mass) whereby the fluid in which the mass is growing can be tested to see if the hybridoma is producing the specific antibody product desired. If it is, the product being produced truly is "monoclonal" since the hybridoma mass producing it was derived from a single hybrid capable of making only one product. The fact that, at the time of such testing, a large number of cells is present due to continuous division and subdivision of the hybrids formed is of no consequence in this case since, given the assumption of only one single hybrid being formed ab initio, all such cells necessarily are identical.

In point of fact, however, the hybridization processes as currently practiced can never provide the assurance that only one single hybrid cell between a myeloma cell and a normal cell is produced. The frequency of myeloma cell/normal cell hybrid formation statistically is on the order of about one to ten such hybrids for every $10^5$ myeloma and normal cells. Accordingly, even in cases where only $10^5$ myeloma cells and normal cells are mixed in some suitable vessel, there can be no assurance that only one hybrid will result. In the case, say, where three such hybrids are formed, the earlier discussed isolation technique will, of course, segregate the mixture of these three hybrids from all other cells. The three hybrids may, however, each be making a different product, since each hybrid may have been derived from three different normal cells. The mixed hybridoma resulting from continued growth of these three hybrids may well be making the desired product but this product may be mixed with e.g., two other different products. Thus, pure monoclonal product is not being produced. It is therefore necessary to grow up each individual cell from the mixture until a homogeneous cell collection is derived making just the desired product. This task is greatly attenuated by the fact that, as earlier noted, by the time the three hybrids per se were isolated from all other cells in the first isolation step to begin with, these hybrids already have divided and sub-divided. Thus, the actual number of cells present at the time it is sought to find just the hybrid making desired product is far more than three, and each such cell must be grown up and tested in the effort to find a hybridoma, derived from a single hybrid cell, and producing the desired monoclonal product. As will be apparent, many of such tests will be redundant since in most cases a particular cell being grown up and tested is merely a cell which has resulted from division of some other cell being tested. Nevertheless, testing of all cells is required.

The required cloning of all these cells adds enormously to the labor, time and cost of the hybridization technique.

It is an object of the present invention to provide a hybridization process wherein it is possible to produce easily isolatable living hybrid cells producing a particular desired product.

This and other objects are achieved by the provision of a process wherein it is arranged that the hybridization of cells occurs in a number of individual, discrete vessels, receptacles or chambers and wherein it is arranged that no more than one hybrid between a continuous dividing cell and a normal cell can occur in any one chamber.

A further feature of the present invention involves the ability to separate from the collection of various vessels, those vessels containing a growing hybrid population making a particular desired product.

According to the most preferred embodiment of the present invention, the provision that no more than one hybrid be formed in any individual chamber is achieved by arranging that in any given chamber, prior to hybridization, there be no more than one pair of a continuous dividing cell and a normal cell. Thus, a given chamber may, for example, contain many continuous dividing cells so long as only one normal cell is present, thereby insuring that only one hybrid between a continuous dividing cell and a normal cell can be formed. Alternatively, a given chamber may contain a number of normal cells so long as only one continuous dividing cell is present, again thereby insuring that only one hybrid between a continuous dividing cell and a normal cell can be formed. In the interests of economizing on materials employed in the process, of course, it is most desirable to arrange that each chamber simply have one continous dividing cell and one normal cell.

When subjected to hybridization conditions, the continuous dividing cell and the normal cell in one or more of the chambers will fuse and form a hybrid cell. By subjecting all the chambers to an appropriate medium (e.g., selective HAT medium), all cells except functional hybrids between a continuous dividing cell and normal cell will die in time (continuous dividing cells by virtue of some pre-arranged genetic deficiency; normal cells by virtue of their inability to continuously divide). Any mass growing in any particular chamber after this treatment necessarily is derived from a hybrid between a continuous dividing cell and a normal cell. More importantly, by reason of the fact that each chamber began with no more than one pair of a continuous dividing cell and a normal cell, the isolated mass necessarily is derived from only one hybrid, capable of producing only one, monoclonal product. Accordingly, all that is required is to test the fluid in which the particular hybridoma is growing to ascertain whether the hybridoma is making the desired product. If it is, no further isolation, cloning, etc. is required.

Although the requirement that no more than one hybrid between a continuous dividing cell and a normal cell be formed in any one chamber is best assured by providing each chamber with no more than one pair of a continuous dividing cell and a normal cell, it is found that this same requirement can be achieved in practice even where each chamber is provided with up to, e.g., 20,000 cells (i.e., wherein there may be as many as 10,000 pairs of a continuous dividing cell and a normal cell) since the frequency of hybrid formation is so low. As will be apparent, this manner of practicing the method of the present invention greatly reduces the number of chambers required to process a large number of cells.

In theory, any suitable vessels or chambers may be employed for practicing the present invention. However, because of the very low frequency of hybrid formation and the need for economizing with respect to production capabilities and material utilization, it is generally required to arrange that somewhere in the order of $10^5$ possibilities for hybridization between a continuous dividing cell and a normal cell occur in any given experiment or production so as to generate at least one hybrid between such cells making a desired product. In the preferred embodiment of this invention, wherein the formation of only a single hybrid per chamber is achieved by providing no more than one pair of a continuous dividing cell and a normal cell in any chamber, $10^5$ individual chambers would therefore be required. Even in the case where each chamber is permitted to have as many as 10,000 such cell pairs, the number of individual chambers required is substantial. To overcome this particular difficulty, the process of the present invention further concerns a practice means for providing a large number of small, discrete individual "chambers" in which such hybridications can occur. An additional benefit of the means provided is the ability to easily isolate those chambers containing growing hybrids from all other chambers.

According to this feature of the present invention, semi-permeable micro-capsules (hereinafter "beads") are employed as the individual discrete chambers in which hybridization may occur. In a method described in further detail hereinafter, a mixture of a large number of continuous dividing cells (hereinafter for illustrative purposes malignant (myeloma) cells) and a large number of normal cells is suspended in a quantity of bead forming medium such that, upon bead formation (and with reference to the earlier-described preferred embodiment of the invention), no more than two cells exist in each formed bead. The two cells may be two myeloma cells, two normal cells or a normal cell and a myeloma cell. When the total collection of beads is subjected to hybridization conditions, one or more of those beads containing a myeloma cell and a normal cell will have formed therein a single hybrid. By thereafter suspending the beads in a suitable medium (e.g., selective HAT medium), the cells of all beads other than those having a functional myeloma/normal hybrid therein will eventually die. Owing to the ever increasing mass (density) of the beads in which living cells (e.g., the myeloma/normal cell hybrids) still remain, it is found that there beads can thereby be "isolated" from all other beads since, in either the HAT medium or any other chosen medium, these more dense beads eventually will sink while the other beads float or remain in suspension. The thus isolated beads, containing growing hybrid cells, can then be tested to see if a desired monoclonal product is being produced.

In a further embodiment of the present invention, the beads so isolated can, in the same step, be further processed so as to isolate only those beads containing a hybridoma producing a particular desired product. This isolation technique, as it pertains to the hybridization process of the present invention, is discussed in further detail hereinafter. The isolation technique per se, being generally applicable to the isolation of any cell producing a desired product, is described in my co-pending U.S. patent applications Serial No. 325,051, filed November 25, 1981 and Serial No. 443,191, filed November 23, 1982, both entitled "Method For Isolating Cells."

According to another feature of the present invention, the use of beads as individual chambers permits an alternative approach to achieving the aim of easily isolating hybridomas producing no more than a single, monoclonal product. In this method, a large number of continuous dividing cells (e.g., myeloma cells) and normal cells are mixed in a single vessel and subjected to hybridization conditions, according to the known hybridication processes. However, as soon as a sufficient time for hybridization to occur has elapsed, and before any hybrids formed have had an opportunity to undergo division, the entire population of cells is subjected to a bead-forming process wherein each individual cell in the population (i.e., each unhybridized normal or myeloma cell, each myeloma/myeloma hybrid, each normal/normal hybrid and each normal/myeloma hybrid) is encapsulated within individual beads. The resultant beads are then subjected to a medium where all but the normal/myeloma hybrid cells die and wherein the beads containing the normal/myeloma hybrids can be separated from all othe beads by specific gravity (the weight of these beads increasing, as earlier described, because of the division and sub-division of the normal/myeloma hybrid). Any such hybridomas isolated in this manner, although now containing a number cells, necessarily were each derived from one normal/myeloma hybrid cell since the beading of all the cells occurred prior to any division or sub-division of the normal/myeloma hybrid cells. Any product produced by the thus-isolated hybridomas necessarily is, therefore, monoclonal produce.

In the hybridization process, there is provided a population of "continuous dividing" cells and a population of "normal" cells, the latter possessing some desirable characteristic sought to be carried through to the hybrid cell where, because of the continuous division characteristics of the continuous dividing parent of the hybrid, the desirable characteristic of the normal cell potentially can be perpetuated.

The continous dividing cell for use in the present invention is any cell which possesses the capability of continous growth in all or particular media or environments and whose ability for such continuous growth can be imparted to a hybrid made therefrom. Typically, the continuous dividing cell is a malignant cell such as a myeloma cell. Alternatively, however, the continuous dividing cell may be a non-malignant cell which, nevertheless, exhibits continuous growth in the presence of particular compounds or media, such as T-cells in the presence of T-cell growth factor. In this latter case, hybrids between a normal cell and a T-cell will exhibit the desired continuous growth when the hybrid cell is in the presence of T-cell growth factor. The continuous dividing cell must also possess the property of enabling its isolation when hybridized to a normal cell. As earlier discussed, this is achieved either by use of a mutant continuous dividing cell which is incapable of growing in a particular medium or through use of a continuous dividing cell which has been overtly pre-treated (e.g., chemically) so as to render it incapable of growth in a particular medium.

For ease of discussion hereinafter, the continuous dividing cell is taken to be a malignant myeloma cell.

Also for purposes of illustration, the population of normal cells is taken to be a population of cells, at least some of which are producing a specific desired antibody sought to be isolated and collected. The remaining cells of the population of normal cells may either be producing no products at all or may be producing products (e.g., antibodies) other than the specific antibody desired.

The population of normal cells as above-defined may be obtained by a number of methods known in the art, such as:

(a) immunizing an organism with the antigen specific to the desired antibody and isolating the cells from the organism (e.g., by collecting the lymphocytes from the immunized organism);

(b) isolating the cells produced by an organism in response to a disease state which exhibits the antigen specific to the desired antibody. For example, a tumor-bearing or tumor-cured organism will have antibody producing cells making antibody against the tumor antigen;

(c)  stimulating normal cells of an organism in vitro with the antigen specific to the desired antibody; and

(d)  treating normal cells of an organism in vitro with a chemical capable of stimulating the normal cells to produce the full complement of antibodies which the cells of that organism are genetically able to produce, including the desired antibody.  Examples of such chemicals are lipopoly-saccharides and various plant lectins.

According to the present invention, individual chambers are filled with myeloma and normal cells in a manner such that, if hybridization between a normal cell and a myeloma cell occurs, only one such hybrid can be formed in any given chamber.  As earlier noted, this can be achieved either by filling each chamber with no more than one pair of a normal cell and a myeloma cell or by filling each chamber with more than one such pair of cells whereby, statistically, only one hybrid will form from all such pairs.  For purposes of describing the invention hereinafter, these chambers are taken to be individual, discrete micro-capsules or beads.  The beads are such that the encapsulating material is semi-permeable, i.e., contains openings or pores of a size sufficiently large to permit various agents and media to contact the cells within the beads but small enough to insure that the cells are retained in the bead.

Hybridization is effected by subjecting the entire bead population to the action of a "cell-fusing" agent, such as polyethyleneglycol (PEG) or inactivated

Sendai virus at appropriate concentrations and conditions. The fusing agent penetrates the bead material and, for those beads containing two cells, causes the cells to stick or attach to one another to form fused cell doublets. A proportion of these doublets will form hybrids. After a sufficient time has been permitted for any hybridizations to occur, all the beads are suspended in a medium in which, owing to the nature of the myeloma cells employed, myeloma cells not hybridized to a normal cell cannot survive. At the same time, normal cells not hybridized to a myeloma cell will die because of their inherent inability to undergo continuous division. As a result, the only beads ultimately containing living cells will be those beads continaing a functional hybrid of a myeloma cell and a normal cell. The cells growing in such beads will be derived from a single hybrid cell (because it is arranged that only one hybrid can form in any given bead) and, hence, will be producing a single monoclonal product (assuming any product is being produced).

In forming the cell-containing beads, a variety of techniques may be employed. In general, the cell population to be encapsulated within beads is uniformly mixed with a suitable gel-forming material such as alginate (which forms a gel in the presence of calcium ions) or agarose (which forms a gel when below a certain temperature) or a mixture of these two materials. Micro-spheres containing cells are formed from these materials by using, e.g., a needle or nozzle to draw off a particular quantity of the mixed suspension of cells and gel-forming material and then deposit the drawn-off materials as droplets into a calcium solution (where alginate is the gel-forming material) or, e.g., cold oil (where agarose is the gel-forming material) to cause gelation of the droplets.

By controlling the number of cells in the cell population, the concentration of gel-forming material mixed therewith and the size of each droplet, each droplet can be arranged to accomodate no more than a given number of cells. Thus, for example, where hybridization is to be effected within the beads per se and where it is desired to insure that no more than one pair of a myeloma cell and a normal cell is present in any bead, the above-noted parameters are controlled to produce micro-spheres which can accomodate no more than two cells. Thus, the gelled micro-spheres may contain no cells at all, or one myeloma cell, or one normal cell, or two myeloma cells or two normal cells or one myeloma cell and one normal cell. Where the requirement of formation of only a single myeloma normal hybrid is to be achieved through provision of, e.g., up to 10,000 pairs of a myeloma cell and a normal cell in each bead, the above-noted parameters are chosen such that the micro-spheres can accomodate no more than, e.g., 20,000 cells. Finally, where hybridization is to be effected in the known prior art manner (i.e., by mixing a large number of myeloma and normal cells in a single vessel under hybridizing conditions) and wherein, as earlier described, each cell of the resultant mixture (including any myeloma normal hybrid cells formed) is to be encapsulated within a bead prior to the occurrence of any cell divisions or subdivisions, the above-noted parameters are chosen such that, preferably, no more than one cell can be accomodated within each microsphere.

After formation of the gelled droplets or micro-spheres, each droplet is provided with a coating of a semi-permeable encapsulating material by way of example, the encapsulating material may be polymerized polylysine. For alginate-based droplets, the polylysine coating can be provided on each droplet by means of the electrostatic attraction between the positively charged alginate gel and the negatively charged polylysine. The polylysine-coated droplets are then subjected to either a calcium-free solution or a solution containing a calcium sequestering agent (e.g., EDTA). When this solution permeates the polylysine coating, it liquifies the alginate gel and the liquified gel diffuses out of the polylysine coating. What remains is a polylysine micro-capsule or bead containing whatever cells were initially present in the gelled alginate droplet. It is these beads which are then subjected to hybridization and/or isolation procedures.

Where the gelled droplets are formed from a material such as agarose, the semi-permeable encapsulating material is provided around each droplet and the thus-coated droplets are heated in an appropriate manner to cause liquification of the agarose. When the liquified agarose diffuses out of the coating of semi-permeable encapsulating material, what remains is a bead containing wheatever cells were initially present in the gelled agarose droplet.

Although it is possible to utilize polylysine as the encapsulating material for droplets formed from agarose, it is generally found that the minimal electrostatic attraction between these two materials makes it somewhat difficult to achieve a uniform coating of polylysine around the agarose droplet. One means of overcoming this difficulty is to form the micro-spheres or droplets from a mixture of agarose and alginate. The amount of alginate employed is chosen to be sufficient to permit polylysine to coat such droplets by reason of its electrostatic attraction for the alginate present in the droplet. Once the coating of polylysine has been so provided, the coated droplets are subjected to solutions and temperatures at which liquification of the alginate and agarose occurs. The liquified materials diffuse out through the polylysine coating and what remains is a cell-containing polylysine bead.

Returning to the general steps of the present invention, after hybridization and suspension of all the beads in a suitable medium (e.g., selective HAT medium), the only beads containing living cells are those containing a hybrid between a myeloma cell and a normal cell. Thus, only in those beads will continuous growth (of a hybridoma) be occurring (because of the removal of the original alginate or agarose, the bead can accomodate many cells and, hence, accomodate a growing, dividing mass of hybrid cells). The continuous growth of the hybridoma will significantly increase the mass of the fixed-volume bead and, as a result, densifies the bead to a point where it can be separated from all other beads using any technique suitable for effecting separations based upon specific gravity differences.

Each of the thus isolated beads (or the hybridomas growing therein) may then be separately tested to determine which, if any, hybridoma is producing the specific product desired. For any positive determination, no further isolation beyond that described above is required since operation of the hybridization process according to the present invention insures that any hybridoma has been derived from a single hybrid cell formed from only one product-producing parent normal cell.

In some cases it will be found that the number of beads containing growing hybridomas is large enough such that, notwithstanding the fact that these beads are easily isolated from all other beads, the testing of each individual bead so isolated to determine if a particular product is being made may be laborious and time-consuming. For this reason, and in view of the fact that beads are employed in the hybridization process per se, a preferred method of practicing the process of the present invention involves utilization of the isolation technique described in my co-pending U.S. applications Serial No. 325,051, filed November 25, 1981 and Serial No. 443,191, filed November 23, 1982 both entitled "Method for Isolating Cells." According to this process, the interaction between a product being produced by a cell(s) (here, a product being produced by a hybridoma) and an affinity material therefor is used to bring about some identifiable and distinguishable physical state only in the immediate vicinity of such cells, thereby permitting the cells to be identified and isolated.

As applied to the hybridization process of the present invention, the foregoing isolation technique may be practiced in a number of ways. Assuming, for purposes of illustration, that the desired product sought is a particular specific monoclonal antibody being produced by one or more hybridomas, the following isolation techniques are suitable.

After isolating from all other beads those beads containing a growing hybridoma, each individual bead is caused to be encapsulated within a second, outer bead, again made of semi-permeable material such a polylysine, agarose or alginate. The volume of the outer bead (the so-called "outer layer" of the overall bead) is arranged to contain therein the antigen specific to the desired antibody. The semi-permeable material of the inner bead (in which inner bead there is contained the growing hybridoma) permits antibody product produced by the hybridoma to diffuse into the outer layer of the overall bead. If this antibody product is the desired product, it will attach to the specific antigen in the outer layer; if this antibody product is not the desired product, it will not exhibit affinity for the specific antigen present in the outer layer. The next step is to add to the entire system a material which can diffuse into the outer layer of every bead but will bind only to desired antibody product or complexes of desired antibody product and its specific antigen. This material (e.g., anti-immunoglobulin which can bid to $F_C$ or $F_{ab}$ regions of immunoglobulin) has affixed to it an enzyme and, as a result of the fact that binding of the material plus enzyme will occur only in those beads making the desired product, it will be seen that, after washing away non-affixed materials, enzyme will remain only in those beads where a hybridoma making the desired product is present. Once the enzyme is affixed in such beads, the total collection of beads can be subjected to any number of environments capable of being selectively converted to an identifiable system in the presence of the enzyme. For example, a colorless material can be added to the entire system, which colorless material is converted to an insoluble colored material in the presence of the enzyme. Since the enzyme is present only in those beads containing a hybridoma making the desired antibody, only those beads will become colored and can thereafter be easily isolated from the remaining colorless beads. Similarly, a non-fluorescent material can be added to all the beads, which material is converted to a fluorescent material in the presence of the enzyme (i.e., only in those beads containing hybridomas making the desired product).

Additional isolation methods employing enzyme affixed only in beads making desired product can be employed. Thus, with all beads in a suitable suspension, the entire bead population can be exposed to a lethal environment which either is rendered non-lethal

0121400

-20-

by the action of the enzyme or is rendered incapable of entering the inner bead by virtue of the presence of enzyme in the outer bead layer. As a result, only those beads containing a hybridoma producing the desired product will survive. Owing to the continued growth of this desired hybridoma within the bead, the desired beads eventually will separate from the remaining beads on the basis of the increased specific gravity of the former.

In another method involving enzyme affixed only in those beads containing hybridoma making desired product, a soluble material can be added to the entire bead population. In the presence of enzyme, this soluble material is converted to an insoluble precipitate and the increased weight of those beads in which the precipitate has formed permits gravimetric separation of these (desired) beads from the remaining beads. In yet another method, a material can be added to a suspension of the entire bead population, which material is converted to one or more gaseous products by the action of the enzyme. The evolution of gases in those beads where the enzyme is present will cause these desired beads to levitate away from the remaining undesired beads.

In a non-enzymatic process for isolating from all hybridoma-containing beads just those beads containing hybridoma making a desired product, the outer bead layer is arranged to have present therein a large number of red blood cells to which the antigen specific to the desired antibody product is affixed. After a suitable incubation period, those beads containing a hybridoma making the desired antibody will have that antibody affixed to the antigen attached to the red blood cells in the outer bead layer. In all other beads, the antigen carried by the red blood cells remains uncomplexed. When a material such as complement is added to the entire system it will, in a known manner, cause the lysis of those red blood cells exhibiting an antigen-antibody complex. The lysed material in these beads escapes through the outer semi-permeable bead surface while, in all other beads, the red blood cells remains intact. As a result, the desired beads (those in which the red blood cells were lysed) are significantly lighter and less dense than the undesired beads and can thereby be isolated by suitable gravimetric techniques.

Based upon the foregoing, illustrations, it can be seen that the process of the present invention, in its preferred mode of practice, provides for a hybridization process in which it is ensured that any isolated growing hybridoma was derived from a single hybrid cell (and thus will be capable of producing only a single product), thereby eliminating the need for resorting to the various cloning and sub-cloning processes in order to make practical use of hybridomas producing a desired product. At the same time, by virtue of the preferred means chosen to effect the hybridization of the present invention (i.e., employing individual, discrete beads as separate reaction chambers), the process lends itself to easy adaptation of my previously-described techniques for isolating particular cells (here, a particular hybridoma) making a specific desired product. As a result, the overall hybridization process, from hybrid formation to isolation of a hybridoma making a particular monoclonal product, is far more efficient and economical than known hybridization/isolation techniques.

As earlier, as noted a basic hybridization technique, known to the art, relies upon a fusing agent (PEG or inactivated Sendai virus) to bring about the cell attachment required as a first step in the formation of hybrid cells. In another form of the hybridization process per se, the reliance upon fusing agents to achieve the initial cell attachments is avoided. This process is decribed in detail in my co-pending U.S. patent application Serial No. 90,130, filed November 1, 1979 and entitled, "A Method For The Production of Monoclonal Antibodies." A European Patent Application corresponding to U.S. application Serial No. 90,130 was published as Specification No. 0028902 on May 20, 1981, and is incorporated herein by reference.

Briefly, the process described in application Serial No. 90,130 was developed in recognition of the fact that even the successful isolation of all hybrids between myeloma cells and normal cells (such as by the use of mutant myeloma cells or chemically pre-treated myeloma cells) still leaves the need to isolate from these hybrids only the hybrids making the specific antibody desired. This is because the fusing agents used in achieving initial cell attachments are random and non-specific; hence, a number of isolatable hybrids of myeloma and normal cells will result, but only a very small proportion of these hybrids will be between a myeloma cell and a normal cell making the specific antibody desired. Even as to the latter, the production of specific antibody by the normal cell may not for some reason have been carried through to the hybrid.

As earlier described in this present application, conducting the hybridization process in a manner which insures that each isolated growing hybridoma was derived from a single myeloma/normal hybrid cell, and further employing in the process the isolation techniques of my earlier application Serial No. 325,051 so as to isolate from these hybridomas just those producing a specific single product, greatly

0121400

-23-

reduces the heretofore laborious task of effecting such isolation, even in the case where fusing agents are employed in the hybridization process. Using the process of my application Serial No. 90,130 to effect hybridization per se can result in yet a further reduction of the efforts required to isolate a particular hybridoma producing a particular product. Thus, according to the process of application Serial No. 90,130, the myeloma cells employed in the hybridization process are provided, either attached to or as part of their surface, with the antigen specific to the specific antibody desired. In the absence of fusing agents, therefore, a mixture of one such myeloma cell and a normal cell in a single (bead) chamber will result in attachment (and eventual possible hybridization) only if the normal cell is producing the specific (desired) antibody which exhibits affinity for the antigenic receptor provided on the myeloma cell. As a result of this technique, the number of desired hybrids (i.e., those actually making the specific antibody desired), relative to all hybrids produced, is very large and such desired hybrids are, therefore, much more easily isolated.

CLAIMS

1.      A method for isolating a living isolatable hybrid of a continuous dividing cell and a normal cell incapable of continuous division, wherein continuous dividing cells and normal cells are mixed together in a reaction vessel under conditions which result in the formation of one or more hybrids of a continuous dividing cell and a normal cell, characterised by effecting such mixing and hybridization in a plurality of discrete, individual reaction vessels containing no more than a predetermined number of continuous dividing cells and normal cells which will insure that a hybrid of a continuous dividing cell and a normal cell can be formed in any particular reaction vessel.

2.      A method according to claim 1 characterised in that prior to hybridization, the individual reaction vessels contain no more than one pair of a continuous dividing cell and a normal cell.

3.      A method according to claim 1 or 2 characterised in that said continuous dividing cell is a malignant cell.

4.      A method according to claim 1,2 or 3 characterised in that said continuous dividing cell, as a result of a genetic deficiency or a chemically-induced inactivation, is incapable of surviving in a predetermined medium, and wherein, after hybridization, said reaction vessels are exposed to said predetermined medium such that, after a predetermined time, the only vessels containing living cells are those containing living hybrid cells of a continuous dividing cell and a normal cell.

5. A method according to claim 4 characterised in that the vessels containing living hybrid cells are further treated so as to isolate those vessels containing living hybrid cells producing a particular product.

6. A method according to any one of the preceding claims characterised in that said reaction vessels containing no more than a predetermined number of continuous dividing cells and normal cells are beads consisting of a semi-permeable surrounding layer, permeable to conditions which result in the formation of a hybrid cell.

7. A method according to claim 6 characterised in that the continuous dividing cells and the normal cells are mixed in the presence of a gel-forming agent under conditions which result in the formation of a plurality of gelled microspheres, each of said microspheres then being surrounded with a semi-permeable surrounding layer, and thereafter the gelled material within said surrounding layer is liquified and removed, thereby forming a plurality of beads consisting of said semi-permeable surrounding layer and, within said surrounding layer, the cell(s), if any, originally present in each particular microsphere.

8. A process according to any one of the preceding claims characterised in that the hybrid is introduced to the individual reaction vessel after said mixing and hybridization is effectuated, but prior to a time when the hybrid has undergone cell division.